# EUROPEAN PATENT APPLICATION

(11) **EP 4 311 554 A1**
(43) Date of publication of application: **31.01.2024**
(21) Application number: 22187868.9
(22) Date of filing: 29.07.2022
(51) Int. Cl.: A61K 38/18, A61K 36/185, A61P 27/02

(54) **COMBINATION FOR USE IN OPHTHALMOLOGY**

(71) Applicant: Dompé farmaceutici S.p.a., 20122 Milano (IT)
(72) Inventor: ROMEO, Tiziana, 60100 L'Aquila (IT); DETTA, Nicola, 80131 Napoli (IT); MANTELLI, Flavio, 67100 L'Aquila (IT); TOMASSETTI, Mara, 80131 Napoli (IT); BIANCHINI, Gianluca, 67100 L'Aquila (IT); LILLINI, Samuele, 80131 Napoli (IT); ARAMINI, Andrea, 67100 L'Aquila (IT); BRANDOLINI, Laura, 67100 L'Aquila (IT); COCCHIARO, Pasquale, 67100 L'Aquila (IT)
(74) Representative: Mauri, Elisabetta Maria Ester

(57) **Abstract**

The present invention relates to a combination of nerve growth factor and sesamin and uses thereof in the treatment of ophthalmic disorders.

## Description

### FIELD OF THE INVENTION

The present invention relates to a combination of nerve growth factor and sesamin and uses thereof in the treatment of ophthalmic disorders.

### STATE OF THE ART

The nerve growth factor (NGF) is a member of the family of evolutionarily well-conserved neurotrophin growth factors that are required for the development and survival of specific neuronal populations and also include brain-derived neurotrophic factor (BDNF), neurotrophin-3 (NT3) and NT4/5.

NGF exerts its activity by interacting with two structurally unrelated cell surface receptors: the high affinity receptor tyrosine kinase A (TrKA) and the low affinity p75 neurotrophin receptor (p75^{NTR}).

NGF, TrKA and p75^{NTR} are broadly expressed in the visual system and play a crucial role both in the trophism of anterior and posterior ocular segment.

For example, NGF modulates the development and differentiation and promotes survival and recovery after injuries of retinal ganglion cells, photoreceptors, optic nerve and visual cortex (Carmignoto et al, J Neurosci.1989, 9: 1263-72; Siliprandi et al, Invest Ophthalmol Vis Sci 1993, 34: 3232-43; Lambiase et al, Graefes Archive for Clinical and Experimental Ophthalmology 1997, 235:780-5, Lambiase et al, Invest. Ophthalmol. Vis. Sci., 2002, 43 (7): 2334-2340, Garcia et al., Cytokine & Growth Factors Reviews 2017, 34: 43-57; Sornelli et al., Molecular Vision 2010; 16:1439-1447; Di Girolamo et al., J Cell Mol. Med 2008, 12(6B): 2799-2811).

In the anterior segment of the eye, TrKA and p75^{NTR} are constitutively expressed in the basal epithelial cells and the stroma of the conjunctiva, as well as the epithelial and endothelial cells of the cornea. Nerve growth factor (NGF) has been demonstrated to maintain ocular surface homeostasis in vitro, ex vivo, and in animal models by acting on epithelial cells health, limbal stem cell differentiation, immune modulation, and tear production (Lambiase et al, Curr Opin Ophthalmol. 2012, 23(4): 296-302; Lambiase et al, Arch Ital Biol. 2011, 149(2): 283-292).

The therapeutic efficacy of NGF in a number of disorders of the cornea, conjunctiva and sclera has been demonstrated in animal models and clinical trials, in particular in phototoxic keratopathy (Rocco ML et al., Graefes Archive for Clinical and Experimental Ophthalmology 2018, 256: 729-738), iatrogenic, immune and neurotrophic corneal epithelial defects and ulcers, scleritis, neurotrophic keratopathy (Lambiase, et al., N Engl J Med 1998, 338: 1174-1180; Lambiase et al., Invest Ophthalmol Vis Sci 2000, 41 (5): 1063-1069; Lambiase et al, Arch Ophthalmol. 2000, 118(10): 1446-1449; Bonini et al., Ophthalmology 2000, 107: 1347-1351; Bonini et al, Ophthalmology 2018, 125(9): 1332-1343), neuropathic corneal pain (Kenyon et al, Investigative Ophthalmology & Visual Science 2021, 62:842), keratoconjunctivitis sicca (Coassin et al., Graefes Arch Clin Exp Ophthalmol Albrecht Von Graefes Arch Klin Exp Ophthalmol 2005, 243(2): 151-155, Sacchetti et al, Br. J. Ophthalmol 2020, 104: 127-135) and Sjögren's syndrome (https://clinicaltrials. gov/ct2/show/NCT05133180).

In the posterior segment of the eye, NGF is expressed in the retinal pigment epithelium, Müller cells, and retinal ganglion cells, while NGF receptors are found on retinal pigment epithelium, photoreceptors, Müller cells, and retinal ganglion cells. NGF receptors have also been shown to be expressed in oligodendrocytes of the optic nerve and several studies have demonstrated that NGF has a neuroprotective effect on neurons in the optic nerve in pathological conditions

The therapeutic efficacy of NGF in retinal and optic nerve disorders have been extensively evidenced, as efficacy has been demonstrated in retinitis pigmentosa (Lenzi et al, Vision Res. 2005, 45(12): 1491-1500; Sacchetti et al, 2017, Current Eye Research, 42:7, 1064-1068), retinal detachment (Sun et al., Ophthalmologica 2008, 222: 58-61), diabetic retinopathy (Ali et al., Diabetes, 2008, 57: 889-898), retinal degeneration subsequent to ischemia (Xien et al., Exp Eye Res 2014, 125: 156-63), phototoxic retinopathy (Rocco et al., Graefes Arch Clin Exp Ophthalmol 2018, 256: 729-738; Garcia et al., J Neurochem 2014, 131(3): 303-13), epiretinal membrane (Minchiotti et al., Retina 2008, 28(4): 628-37), macular hole (Zhang et al., BMC Ophthalmol 2019, 19(1): 130), macular degeneration (Lambiase et al., Ann Ist Super Sanità 2009, 45 (4): 439-442), optic neuropathies (Mesentier-Louro et al., Mol Neurobiol. 2019, 56(2): 1056-1069; Guo et al Sci Rep 2020, 10: 3375), optic gliomas (Falsini et al, Brain J Neurol 2016, 139(Pt2): 404-414), and glaucoma (Lambiase et al, Proc Natl Acad Sci USA 2009, 106(32): 13469-13474; Lambiase et al, Graefes Arch Clin Exp Ophthalmol Albrecht Von Graefes Arch Klin Exp Ophthalmol 1997, 235(12): 780-785).

Pharmacokinetic studies have shown that after topical administration of NGF, at a concentration of 200 µg/mL, high levels of the protein are detected in all ocular tissues, included the retina andoptic nerve, showing a peak increase 6 hours after administration (Lambiase et al, IOVS 2005, Vol. 46 (10): 3800-3806). This route of administration is therefore suitable also for the treatment of pathologies of the retina and optic nerve. The topical administration of NGF on the ocular surface has been demonstrated in clinical trials to be safe. However, the primary side effect reported by patients after topical ocular administration of NGF in clinical trials or using NGF for approved indications is eye discomfort, including eye pain, eye or eyelid irritation, eye pruritus and an abnormal sensation in the eye. This side effect has been suggested to be associated at least in part to the therapeutic activity of NGF, since the restoring eye innervation and sensitivity can be associated with increased ocular surface symptoms. However, this has not been yet fully investigated.

Although the above side effect is mild and transient, in a few cases it leads to discontinuation of the treatment and creates distress to the patients. Furthermore, it may interfere with the evaluation of the treatment of ocular discorders that are characterized by similar symptomatology, such as neuropathic corneal pain, dry eye and ocular automimmune diseases, where the patients experience eye pain, burning and stinging as a symptom of the pathology. In these cases, the therapeutic effect of NGF could be masked or substantially diminished by the above side effects. Furthermore, this side effect is particulary enhanced when the topical application of NGF is used for the treatment of pathologies of the posterior segment of the eye and therefore high concentrations of NGF are used.

It is thus a felt need to develop an ophthalmic composition of NGF that does not produce ocular discomfort.

Sesamin, having IUPAC name 5,5'-[(1*S*,3a*R*,4*S*,6a*R*)-Tetrahydro-1*H*,3*H*-furo[3,4-*c*]furan-1,4-diyl]bis(2*H*-1,3-benzodioxole), is a lignan abundantly present in sesame seeds and sesame oil, which has been described to possess a number of diverse physiological effects and is widely used as food supplement, especially in Asia. For example, sesamin has been described to exert hypoglycemic effects, reactive oxygen species (ROS) scavenging activity, antioxidant and anti-inflammatory activity, anxiolytic effect and to inhibit diabetes-associated cognitive decline (Farbood et al, Life Sciences 2019, 230: 169-177; Guo et la, Nutritional Neuroscience 2016: 19(6): 231-236).

### SUMMARY OF THE INVENTION

The present inventors have now surprisingly found that when sesamin is administered in combination with nerve growth factor (NGF), it is able to counteracts the above side effects associated to topical application on the ocular surface.

According, a first object of the invention is a combination of nerve growth factor (NGF) and sesamin.

A second object of the invention is an ophthalmic composition comprising nerve growth factor (NGF) and sesamin.

A third object of the invention is a kit comprising i) an ophthalmic composition containing nerve growth factor (NGF) and ii) an ophthalmic composition containing sesamin.

A fourth object of the invention is a method for the treatment of an ophthalmic disorder comprinsing administering to a patient in need thereof the above combination or ophthalmic composition(s).

### BRIEF DESCRIPTION OF THE FIGURES

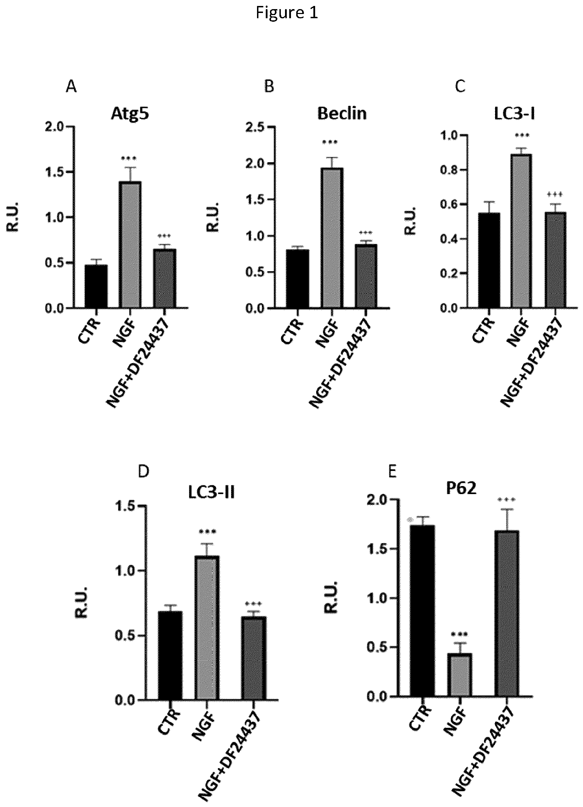

Figure 1 shows the levels of Atg5 (panel A), Beclin (Panel B), LC3-I (panel C), LC3-II (panel D) and P62 (panel E), in control cells (CTR) and in DRGs after treatment with NGF (NGF), or NGF in combination with sesamin (NGF + DF24437), measured as described in Example 1 and expressed as relative optical units.

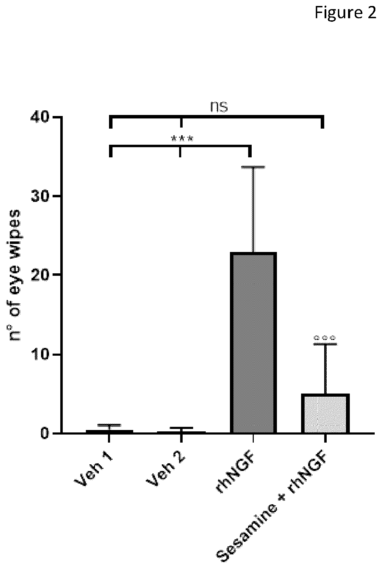

Figure 2 shows the number of eye wipes after ocular administration of vehicle 1 (Veh 1), vehicle 2 (Veh 2), rhNGF in vehicle 1 (rhNGF) and rhNGF and sesamin in vehicle 2 (Sesamin + rhNGF), as described in Example 2. Data are shown as mean ±SEM (n=4-8). ***p<0.001 vs vehicle 1 and vehicle 2 groups; °°° p<0.001 vs rhNGF group.

### DETAILED DESCRIPTION OF THE INVENTION

The present invention is based on the observation that sesamin has the ability to mitigate the ocular discomfort induced by administration to the ocular surface of nerve growth factor.

Accordingly, a first object of the invention is a combination of nerve growth factor (NGF) and sesamin.

Preferably, said combination is in a form suitable for topical administration to the eye. According to one embodiment, said combination is contained in a single ophtalmic composition.

Accordingly, a second object of the present invention is an ophthalmic composition comprising nerve growth factor (NGF) and sesamin.

According to an alternative embodiment, said combination is a combination of two ophtalmic compositions, one comprising nerve growth factor (NGF) and the other comprising sesamin, to be administered simultaneously or sequentially.

Accordingly, a third object of the invention is a kit comprising:
a) an ophthalmic composition comprising nerve growth factor (NGF), and
b) an ophthalmic composition comprising sesamin.

Preferably, said nerve growth factor (NGF) is human nerve growth factor (hNGF).

Preferably, said NGF has the aminoacid sequence of SEQ. ID NO.1 below

SEQ. ID NO.1:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

Alternatively, said NGF has the aminoacid sequence of SEQ ID NO. 2 below:

SEQ ID NO. 2:
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

Alternatively, said NGF is a mixture of NGFs having sequences of SEQ ID NO. 1 and SEQ ID NO. 2.

Preferably, said NGF is a recombinant hNGF (rhNGF), produced by recombinant DNA technology. Methods of producing rhNGF are known to the person skilled in the art, for example those described in WO0022119A1 and WO2013092776A1.

Preferably, said NGF has a purity higher than 70%, more preferably higher than 80%, higher than 90%, higher than 95%, higher than 98% or higher than 99%. The purity of NGF may be determined by conventional means known to those skilled in the art, for example by HPLC analysis.

Preferably, said sesamin is in form of a purified compound, preferably having a purity higher than 70%, more preferably higher than 80%, higher than 90% or higher than 95%. The purity of sesamin may be determined by conventional means known to those skilled in the art, for example by HPLC analysis.

According to an alternative embodiment, preferably said sesamin is in a non-purified form, as a component of sesame oil.

Preferably, the ophthalmic composition according to the second object of the invention or the ophthalmic compositions of the kit according to the third object of the invention further comprise at least one ophtalmically acceptable excipient.

Preferably, said ophthalmically acceptable excipient is selected from ophthalmically acceptable viscosity enhancers, penetration enhancers, buffering agents, osmolarity regulators, preservatives, antioxidants, surfactants, emulsifying agents and polyethylene glycols.

Viscosity enhancers are preferably selected from polyvinylpyrrolidone, cellulose ethers, preferably hydroxymethylcellulose, hydroxyethylcellulose (HEC), hydroxypropylmethylcellulose (HPMC) and methylcellulose, and gelling agents, preferably gellan gum, xanthan gum and carbopol-974. A preferred viscosity enhancer according to the invention is hydroxypropylmethylcellulose.

Penetration enhancers are preferably selected from cyclodextrins, chelating agent, crown ethers, bile acids and bile salts.

Buffering agents are compounds capable of providing and maintaining the pH of the ophtalmic composition at values compatible for use in the eye, preferably at a pH comprised between 6.5 and 8. The preferred buffer is phosphate buffer, but other buffers capable of maintaining the pH within the desired range, suitable for ophthalmic use, are also included.

Osmolarity regulators are salts able to make the ophtalmic composition isotonic with ocular fluids. The preferred salt is sodium chloride (NaCl) but other ophthalmically acceptable salts may be used, such as for instance potassium chloride (KCI), calcium chloride (CaCl₂) and magnesium chloride (MgCl₂) and their admixtures.

Preservatives are preferably selected from quaternary ammonium compounds such as benzalkonium chloride, cetyltrimethylammonium bromide and cetylpyridinium chloride. Surfactants and emulsifying agents are preferably selected from linoleic acid sodium salt, polysorbates, preferably Tween 80, and poloxamers, preferably Pluronics F68. Preferably, the ophthalmic composition according to the second object of the invention or the ophthalmic compositions of the kit according to the third object of the invention comprise one or more excipients selected from trehalose, mannitol, cellulose ethers, buffering agents, polyethylene glycols and antioxidant compounds.

According to a preferred embodiment, the ophthalmic composition according to the second object of the invention or the ophthalmic compositions of the kit according to the third object of the invention comprise as excipients trehalose, mannitol, a cellulose ether, a buffering agent and a polyethylene glycol.

According to another prefererred embodiment, the ophthalmic composition containing sesamin of the kit according to the third object of the invention, comprises as excipients a buffering agent and a polyethylene glycol.

The combination according to the first object of the invention, the ophthalmic composition according to the second object of the invention or the ophthalmic compositions of the kit according to the third object of the invention are in a form suitable for topical administration to the ocular surface.

The ophthalmic composition according to the second object of the invention or the ophthalmic compositions of the kit according to the third object of the invention are preferably in form of a ready-to-use liquid ophthalmic formulation or of a powder or granulate formulation, preferably a lyophilized powder, to be dissolved at the moment of use in a suitable vehicle to form the liquid ophthalmic formulation.

According to one preferred embodiment, said ophthalmic composition according to the second object of the invention is in form of a liquid ophthalmic formulation.

According to an alternative preferred embodiment, said ophthalmic composition according to the second object of the invention is in form of a powder formulation. According to one preferred embodiment, in the kit according to the invention, at least one of said ophthalmic compositions a) and b) is in form of a liquid ophthalmic formulation. Preferably, in the kit according to the invention, said ophthalmic composition comprising nerve growth factor (NGF) and said ophthalmic composition comprising sesamin are both in form of a liquid ophthalmic formulation.

Preferably, in the kit according to the invention, said ophthalmic composition comprising nerve growth factor (NGF) and said ophthalmic composition comprising sesamin are one in form of a liquid ophthalmic formulation and the other in form of a powder formulation. According to an alternative preferred embodiment, in the kit according to the invention, said ophthalmic composition comprising nerve growth factor (NGF) and said ophthalmic composition comprising sesamin are both in form of powder formulations.

By "liquid ophthalmic formulation" according to the present invention it is meant a formulation suitable to be used for application at the surface of the eye and to be dispensed in small volume units, such as drops, from a dropper.

Preferably, said liquid ophthalmic formulation comprises NGF and/or sesamin and one or more of said ophthalmically acceptable excipients, as above described, dissolved or suspended in a liquid vehicle, preferably an aqueous liquid vehicle.

Preferably, said liquid ophthalmic formulation has an osmolarity between 200 and 380 mOsmol/kg, more preferably between 250 and 330 mOsmol/kg, even more preferably between 280 and 320 mosm/Kg.

Preferably, said liquid ophthalmic formulation has a pH between 6.5 and 8, more preferably between 6.8 and 7.5, even more preferably between 7 and 7.4.

When said liquid ophthalmic formulation comprises NGF, as above described, this is present in said liquid ophthalmic formulation at a concentration preferably between 5 µg/ml and 300 µg/ml. When said ophthalmic formulation is for use in the treatment of ophthalmic disorders affecting the anterior portion of the eye, preferably cornea, conjunctiva and sclera, said NGF is present in said liquid ophthalmic formulation at a concentration preferably between 10 µg/ml and 50 µg/ml, more preferably of 10, 20, 30 or 40 µg/ml.

Alteratively, when said ophthalmic formulation is for use in the treatment of ophthalmic disorders affecting the posterior portion of the eye, preferably retina or optic nerve, said NGF is present in said liquid ophthalmic formulation at a concentration preferably between 50 µg/ml and 300 µg/ml, more preferably between 100 µg/ml and 250 µg/ml, even more preferably of 150 or 200 µg/ml.

When said liquid ophthalmic formulation comprises sesamin, this is present in said liquid ophthalmic formulation at a concentration preferably between 2 and 20 µg/ml, more preferably between 2 and 15 µg/ml, even more preferably between 3 and 5 µg/ml. Accordingly, when the sesamin is present in the formulation in unpurified form in sesame oil, the amount of sesame oil present in the liquid ophthalmic formulation will be such as to result in the above concentration of sesamin.

Preferably, when said liquid ophthalmic formulation comprises sesamin, the vehicle is an aqueous vehicle further comprising an ophtalmically acceptable organic solvent, preferably dimethyl sulfoxide (DMSO).

Preferaby, said DMSO is present in said liquid ophthalmic formulation at a concentration between 0.1 to 1.2% v/v, more preferably between 0.2 and 0.9% v/v, even more preferably of 0.2% or 0.6% v/v.

Preferably, the liquid ophthalmic formulation of the invention comprises one or more excipients selected from trehalose, mannitol, a cellulose ether, a buffering agent, a polyethylene glycol and, optionally, an antioxidant compound.

According ot one embodiment, the liquid ophthalmic formulation of the invention comprises trehalose, mannitol, a cellulose ether, a buffering agent, a polyethylene glycol and, optionally, an antioxidant compound

According to another embodiment, the liquid ophthalmic formulation of the invention comprises a buffering agent and a polyethylene glycol. This embodiment is particularly preferred for the composition containing sesamin of the kit according to the third object of the invention.

Preferably, said polyethylene glycol has a molecular weight between 4000 and 8000 g/mol, more preferably between 5000 and 7000 g/mol, more preferably between 5500 and 6500 g/mol, even more preferably of 6000 g/mol. Preferably, when the polyethylene glycol has a molecular weigh of 6000 g/mol (PEG6000) it is present in the liquid ophthalmic formulation at a concentration comprised between 5 and 15 mg/ml, preferably between 8 and 12 mg/ml.

Preferably, said antioxidant compound is selected from L-methionine and cysteine. Preferably, the antioxidant compound L-methionine is present in said liquid ophthalmic formulation at a concentration comprised between 0.005 and 0.02 mg/ml, more preferably of 0,01 mg/ml.

Preferably, trehalose is present in said liquid ophthalmic formulation at a concentration comprised between 30 and 60 mg/ml, more preferably between 40 and 55 mg/ml, more preferably between 45 and 50 mg/ml.

Preferably, mannitol is present in said liquid ophthalmic formulation at a concentration comprised between 5 and 20 mg/ml, more preferably between 10 and 15 mg/ml. Preferably, the concentration of the cellulose ether in said liquid ophthalmic formulation is such as to obtain a kinematic viscosity of about 1-25 cSt determined at 25 °C using a capillary viscometer.

Preferably, said cellulose ether is hydroxypropylmethylcellulose, more preferably at a concentration comprised between 0.5 and 2 mg/ml, preferably between 0.8 and 1.2 mg/ml.

Preferably, the buffering agent in said liquid ophthalmic formulation has a composition and concentration such as to obtain a pH of the liquid ophthalmic formulation between between 6.5 and 8, more preferably between 6.8 and 7.5, even more preferably between 7 and 7.4. Preferably, said buffering agent is phosphate buffer.

Preferably, the ophthalmic composition according to the second object of the invention or the ophthalmic composition of the kit according to the third object of the invention in form of a powder is obtained by lyophilization of the above described liquid ophthalmic formulation.

As discussed above and demonstrated in the experimental section, the present inventors have found that sesamin improves tolerability of NGF when applied topically to the eye fro the treatment of ocular conditions. In particular, eye discomfort associated to administration of NGF is greatly reduced in the presence of sesamin.

Accordingly, a further object of the invention is the above described combination, ophthalmic composition or kit for use in the treatment of an ocular disorder. Preferably, said use is by topical administration.

Preferably, said ocular disorder is a corneal, conjunctival, scleral, retinal or optic nerve disorder.

More preferably said ocular disorder is selected from toxic and phototoxic keratopathy, iatrogenic, immune and neurotrophic corneal epithelial defects and ulcers, neurotrophic keratopathy, neuropathic corneal pain, keratoconjunctivitis sicca, Sjögren's syndrome, retinitis pigmentosa, retinal detachment, retinal degeneration subsequent to ischemia, phototoxic retinopathy, epiretinal membrane, macular hole, macular degeneration, optic neuropathies, glaucoma, scleritis and episcleritis.

The number of administrations per day and the number of drops used in each administration of the ophthalmic composition of the invention will vary with the concentration of NGF and the characteristics of the patients such as age, type and severity of the condition, duration of the treatment, presence of concurrent therapies, and like factors that are within the knowledge and expertise of the skilled person. Usually, each administration consists in the instillation of one or two drops of the ophthalmic composition according to the second object of the invention or of each of the composition(s) of the kit according to the third object of the invention in form of liquid ophthalmic formulation.

The ophtalmic compositions of the kit according to the third object of the invention are administered to the patient separately.

In order to prevent the ocular discomfort induced by topical administration of NGF, the ophtalmic compositions of the kit according to the third object of the invention are preferably administered substantially simultaneously or sequentially in any order, with an interval of no more than 5 minutes, preferably of no more than 2 minutes, one from the other.

### EXPERIMENTAL PART

### Example 1

Autophagy is a a critical adaptive mechanism triggered by stress or damage. It occurs at low basal levels in all cells, but can be activated by numerous stress stimuli in pathological conditions such as starvation, inflammation and by pharmacological agents.

It is a self-digestion process involved in protein and organelle degradation to improve the survival rate of cells in a stressful environment. There are three types of autophagy: macroautophagy, microautophagy, and chaperonemediated autophagy (CMA) (Glick et al, J. Pathol. 2010, 221: 3-12). Alterations in macroautophagic activities in the dorsal root ganglia (DRG) has been demonstrated in injury conditions (Guo et al, Neurosci. Lett. 2015, 599: 158-163). At the beginning of the macroautophagic process, Beclin-1 (the upper stream proteins of the macroautophagy process) at the endoplasmic reticulum respond to the stress signaling pathway and initiate phagophore formation. In the next step, autophagy-related protein 5 (Atg5), microtubule associated protein 1A/1B-light chain 3 (LC3)-II, and p62, are involved in autophagosome formation (Glick et al, J. Pathol. 2010, 221: 3-12). Then, autophagosomes fuse with lysosomes, leading to autolysosome formation and further degradation. When macroautophagic activities increase, the expression levels of LC3-II, Beclin-1, and Atg5 increase, but p62 levels (delivering ubiquitinated cargoes to autophagosomes) decrease.

The effect of NGF on autophagy in the presence and absence of sesamin was assessed on Dorsal Root Ganglia (DRG) neurons, involved in the development of NGF sensory side effects, by analysing the impact of the different treatments on the levels of the above proteins involved in autophagy pathway.

F11 cells were cultured in Dulbecco's modified Eagle's medium (DMEM, Cat#D6546; Sigma-Aldrich, St. Louis, MO, USA), with 10% fetal bovine serum, 2mM glutamine (Sigma-Aldrich, USA). Cells were incubated at 37 °C in a humidified atmosphere with 5% CO2.

Cells were then seeded at 7000 cells/cm² and were maintained in a low serum medium for almost 2 weeks to induce differentiation into DRGs. The complete composition of the low serum medium was: DMEM supplemented 2 mM glutamine, 1% FBS (Sigma-Aldrich, St. Louis, MO, USA). The medium was replaced every 2 days. Immunofluorescence was used to confirm cell differentiation (data not shown).

F11 cells maintained in 10% FBS medium were used as control (control, undifferentiated). Morphological and functional analyses were performed after two weeks of treatment with low serum, at complete differentiation of the cells. The undifferentiated control cells underwent the same analysis at 7 days to avoid dramatic cell death due to confluence. The fully differentiated cells were treated with 1µM rhNGF alone or in combination with 30 nM Sesamin, for 24h.

Control and treated cells were collected and lysed in ice-cold RIPA buffer (Thermo Scientific, USA) with freshly added protease and phosphatase inhibitors. Protein lysates (30 µg) were separated on 4%-12% precast gel using MOPS or MES buffers (Thermo Scientific, USA) depending on the size of the proteins of interest. Then proteins were electroblotted on polyvinyldifluoride membrane (PVDF; Sigma, St Loius, USA) and nonspecific binding sites were blocked in Blocking buffer (Thermo Scientific) for 15 minutes at RT.

Afterward, membranes were incubated overnight at 4° C with the following primary antibodies, diluted in Blocking buffer: rabbit anti-Atg5 1:1000 (Abcam, ab228668, UK), rabbit anti-LC3 1:2000 (Abcam, ab192890, UK), rabbit anti-Beclin 1:2000 (Abcam, ab207612, UK), rabbit anti-P62 1:500 (Abcam, ab91526, UK), rabbit anti-vinculin 1:1000 (Abcam, ab219649, UK) and HRP-conjugated Actin 1:1000 (Cell Signaling, #5125, USA). As secondary antibodies, peroxidase-conjugated anti-rabbit IgG 1:10000 (Abcam, UK) were used. Immunoreactive bands were visualized by ECL (Thermo Scientific, USA), according to the manufacturer's instructions. The relative optical densities of the immunereactive bands were determined and normalized with respect to those of the anti-Vinculin or anti-Actin (depending on the molecular weight of the analyzed protein and the gel/buffer used), using ImageJ software. The phosphorylated forms were normalized upon the total form.

Figure 1 shows the results of the analysis, where the amounts of Atg5, Beclin-1 and LC3-I and LC3-II detected after each treatment are expressed as relative optical density units (RU). As can be seen from the Figure, treatment with NGF alone significantly increased the levels of Atg5 and Beclin, that are essential for the execution of autophagy, while decreased the level of P62 (autophagy-specific substrate). Moreover, the combination of increased levels of LC3-I and LC3-II, the microtubule-associated proteins, and the reduction of the autophagy substrate p62 are consistent with active autophagy-mediated protein degradation. When sesamin was combined with NGF, autophagy was significantly reduced. The results obtain demonstrate that sesamin can inhibit the stress response induced by the treatment of the cells with NGF.

### Example 2

An eye wiping test was carried out to evaluate ocular discomfort induced by ocular administration of rhNGF alone or in combination with Sesamin.

The experiments were performed on male Sprague-Dawley rats (300-320 g), Charles River, Italy) housed in the animal care facility of the Department of Pharmacy, University of Naples. Food and water were available ad libitum.

Rats were divided in 4 experimental groups:

### Group 1- Vehicle 1 control group.

Animals were treated with Vehicle 1 having the following composition in water for injection (WFI) :

| Ingredient | Concentration |
|---|---|
| Trehalose dihydrate | 47.03 mg/ml |
| Mannitol | 12.22 mg/ml |
| Na2HPO4 anhydrous | 2.87 mg/ml |
| Na2H2PO4 dihydrate | 1.22 mg/ml |
| HPMC | 1 mg/ml |
| PEG 6000 | 10 mg/ml |

### Group 2- Vehicle 2 control group.

Animals were treated with Vehicle 2 having the following composition in water for injection (WFI):

| Ingredient | Concentration |
|---|---|
| Trehalose dihydrate | 47.03 mg/ml |
| Mannitol | 12.22 mg/ml |
| Na2HPO4 anhydrous | 2.87 mg/ml |
| Na2H2PO4 dihydrate | 1.22 mg/ml |
| HPMC | 1 mg/ml |
| PEG 6000 | 10 mg/ml |
| DMSO | 0.6% |

### Group 3- rhNGF treated group:

Animals were treated with 20 µg/ml of rhNGF in Vehicle 1 (n=8);

### Goup 4- rhNGF+sesamin treated group:

Animals were treated with 20 µg/ml of rhNGF and 3.5 µg/ml sesamin in Vehicle 2 (n=8) All compositions tested were prepared immediately before use from lyophilized placebo or rhNGF reconstituted in a volume of 1mL of the vehicle.

Animal care was in compliance with Italian regulations on protection of animals used for experimental and other scientific purposes, as well as with European Economic Community regulations (OJ of E.C. L 135 358/1 12/18/1986).

The treatments described above were administered as eye drops 1 time per day, in a volume of 10µL. Following the application, the eye wiping test was performed.

Animals were placed on a 50 × 50cm table for 10 min habituation period. 10µL of solutions were applied into the left eye of animals and the number of eye wipes was counted for 30 min.

Th results of the test are shown in Figure 2.

As can be seen from the results obtained, the rats treated with rhNGF alone showed a significant increase of the number of eye wipes compared to those treated with Vehicle 1 or Vehicle 2 (***p<0.001; respectively), while mice receiving rhNGF in combination with sesamin developed a modest or no significant increase of the numbers of eye wipes with respect to Vehicle 1 or vehicle 2. Moreover, significant difference was observed between rhNGF and Sesamin + rhNGF groups (°°°p< 0.001).

Therefore, sesamin can relieve the eye discomfort induced by rhNGF.

All data are presented as the mean ± SEM. Analysis of data were conducted using GraphPad Prism 8. Statistical analysis was performed by two-way ANOVA followed by Tukey's test for multiple comparisons, as appropriate. Statistical significance was set at *p<0.05.

### Example 3

### Preparation of formulations according to the invention

### Formulation 1 - liquid ophthalmic formulation containing NGF and sesamin

Weight and solubilize in the appropriate volume of WFI the following excipients: Thehalose dihydrate (5.04% w/v), Mannitol (1.31%, w/v), Na2HPO4 anhydrous (0.3065% w/v), NaH2PO4 * H2O (0.116 % w/v), hydroxypropylmethylcellulose (0.107%, w/v), PEG 6000 (1.07%, w/v).

Wait for the complete solubilization of excipients.

Separately, solubilize about 2-4 mg sesamin powder in 1, 2 or 3 mL DMSO and shake the solution to obtain the complete solubilization of the powder. Add the solubilized sesamin in solution A and stir for 5 minutes. Add WFI until the defined volume (500 mL) and filter with 0.22 um filter.

Add the rhNGF at the defined concentration.

### Formulation 2: liquid ophthalmic formulation containing NGF

Weight and solubilize in the appropriate volume of WFI the following excipients: thehalose dihydrate (5.04% w/v), Mannitol (1.31%, w/v), Na2HPO4 anidro (0.3065% w/v), NaH2PO4 * H2O (0.116 % w/v), (hydroxypropylmethylcellulose (0.107%, w/v), PEG 6000 (1.07%, w/v) in WFI. Wait for the complete solubilization of excipients. Check the pH (about 7.3). Add WFI until the defined volume (e.g. Lt). Filter the formulation with 0.22 µm filter. Add the rhNGF at the defined concentration.

### Formulation 2: liquid ophthalmic formulation containing sesamin

Solution A: Weight and solubilize in the appropriate volume of WFI the following excipients: thehalose dihydrate (2.52% w/v)), Mannitol (0.65%, w/v), hydroxypropylmethylcellulose (0.0535%, w/v), PEG 6000 (0.535%, w/v) in WFI. Wait for the complete solubilization of excipients. Add WFI until the defined volume (e.g. 1 Lt). Solution B: Solubilize about 9 mg sesamin powder in 10 mL of DMSO and shake the solution to obtain the complete solubilization of the powder.

Add 300 µL of solution B in 50 mL solution A. Stir for 5 minutes. Aliquote the final formulation in glass vials to perform the lyophilization process.

After the lyophilization process, reconstitute the cake with WFI and filter it with 0.22 µm.

### Formulation 3: liquid ophthalmic formulation containing sesamin

Add 50-100 µL of sesame oil in 5 mL WFI and homogenize at 15000 rpm for 5 minutes, by a homogenizer Ultraturrax t25 basic (IKA), using 8G probe.

Add PEG 6000 (final concentration 0.25%) in the first emulsion and homogenize for additional 5 minutes at 15000 rpm.

### Formulation 4: liquid ophthalmic formulation containing sesamin

Weight about 45 mg micronized sesamin powder (sesamin+PEG6000 or sesamin+Trehalose or sesamin+Mannitol) and solubilize in 500 mL WFI (or WFI+0.6%DMSO). Stir the formulation for 10 minutes and filter with 0.22 µm filter.

## Claims

1. A combination of nerve growth factor (NGF) and sesamin, suitable for topical administration to the eye.

2. An ophthalmic composition comprising nerve growth factor (NGF) and sesamin.

3. A kit comprising:
a) an ophthalmic composition comprising nerve growth factor (NGF), and
b) an ophthalmic composition comprising sesamin.

4. A combination according to claim 1, an ophthalmic composition according to claim 2 or a kit according to claim 3, wherein said NGF has the aminoacid sequence of SEQ. ID NO.1 or of SEQ ID NO. 2.

5. A combination according to claim 1 or 4, an ophthalmic composition according to claim 2 or 4 or a kit according to claim 3 or 4, wherein said NGF is recombinant human NGF.

6. A combination according to claim 1, 4 or 5, an ophthalmic composition according to claim 2, 4 or 5 or a kit according to claim 3, 4 or 5, wherein said NGF has a purity higher than 70%, more preferably higher than 80%, higher than 90%, higher than 95%, higher than 98% or higher than 99%

7. A combination as claimed in claim 1, 4, 5 or 6, an ophthalmic composition according to claim 2, 4, 5 or 6 or a kit according to claim 3, 4, 5 or 6, wherein said sesamin is a purified compound.

8. A combination, ophthalmic composition or kit as claimed in claim 6, wherein said sesamin has purity higher than 70%, more preferably higher than 80%, higher than 90% or higher than 95%.

9. An ophthalmic composition according to claim 2, 4, 5, 6, 7 or 8 or a kit according to claim 3, 4, 5, 6, 7 or 8 wherein said composition comprise one or more excipients selected from trehalose, mannitol, cellulose ethers, buffering agents, polyethylene glycols and antioxidant compounds.

10. An ophthalmic composition as claimed in claim 2, 4, 5, 6, 7, 8 or 9 in form of a liquid ophthalmic formulation.

11. A kit as claimed in claim 3, 4, 5, 6, 7, 8 or 9, wherein at least one of said ophthalmic compositions a) and b) is in form of liquid ophthalmic formulation.

12. An ophthalmic composition as claimed in claim 10, wherein said liquid ophthalmic formulation comprises NGF at a concentration between 5 µg/ml and 300 µg/ml.

13. A kit as claimed in claim 11, wherein said ophthalmic composition a) in form of liquid ophthalmic formulation comprises NGF at a concentration between 5 µg/ml and 300 µg/ml.

14. An ophthalmic composition as claimed in claim 10 or 12, wherein said liquid ophthalmic formulation comprises sesamin at a concentration between 2 and 20 µg/ml.

15. A kit as claimed in claim 11 or 13, wherein said ophthalmic composition b) in form of liquid ophthalmic formulation comprises sesamin at a concentration between 2 and 20 µg/ml.

16. A combination as claimed in claim 1, 4, 5, 6 or 7, an ophthalmic composition according to claims 2, 4, 5, 6, 7, 8, 9, 10, 12 or 14 or a kit according to claims 3, 4, 5, 6, 7, 8, 9, 11, 13 or 15, for use by topical administration in the treatment of an ocular disorder.

17. A combination, ophthalmic composition or kit for use as claimed in claim 16, wherein said ocular disorder is selected from a corneal, conjunctival, scleral, retinal or optic nerve disorders.

18. A combination, ophthalmic composition or kit for use as claimed in claim 16 or 17, wherein said ocular disorder is selected from toxic and phototoxic keratopathy, iatrogenic, immune and neurotrophic corneal epithelial defects and ulcers, neurotrophic keratopathy, neuropathic corneal pain, keratoconjunctivitis sicca, Sjögren's syndrome, retinitis pigmentosa, retinal detachment, retinal degeneration subsequent to ischemia, phototoxic retinopathy, epiretinal membrane, macular hole, macular degeneration, optic neuropathies, glaucoma, scleritis and episcleritis.
